(19) Europäisches Patentamt
European Patent Office
Office européen des brevets

(11) Veröffentlichungsnummer : **0 148 400**
**B1**

(12) # EUROPÄISCHE PATENTSCHRIFT

(45) Veröffentlichungstag der Patentschrift : **06.05.87**

(51) Int. Cl.⁴ : **C 07 C125/06**, C 08 K 5/18

(21) Anmeldenummer : **84114603.8**

(22) Anmeldetag : **01.12.84**

(54) **In ungesättigte Harze einpolymerisierbare Arylamin-Derivate, ihre Herstellung und ihre Verwendung als Härtungsbeschleuniger.**

(30) Priorität : **13.12.83 DE 3345104**

(43) Veröffentlichungstag der Anmeldung : **17.07.85 Patentblatt 85/29**

(45) Bekanntmachung des Hinweises auf die Patenterteilung : **06.05.87 Patentblatt 87/19**

(84) Benannte Vertragsstaaten : **AT DE FR GB IT**

(56) Entgegenhaltungen :
**EP-A- 0 037 314**
**DE-A- 1 643 972**
**DE-A- 2 454 253**
**DE-B- 2 308 036**

(73) Patentinhaber : **BAYER AG**
**Konzernverwaltung RP Patentabteilung**
**D-5090 Leverkusen 1 Bayerwerk (DE)**

(72) Erfinder : **Meier, Helmut-Martin, Dr.**
**Wodantal 28**
**D-4320 Hattingen (DE)**
Erfinder : **Dhein, Rolf, Dr.**
**Deswatinesstrasse 30**
**D-4150 Krefeld (DE)**
Erfinder : **Winkel, Jens, Dr.**
**Hahnenweg 6**
**D-5000 Koeln 80 (DE)**
Erfinder : **Klein, Gerhard, Dr.**
**von-Flotow-Strasse 7**
**D-4019 Monheim (DE)**
Erfinder : **Klöker, Werner, Prof. Dr.**
**Deswatinesstrasse 26**
**D-4150 Krefeld (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 148 400 B1

**Beschreibung**

Die Erfindung betrifft N,N-disubstituierte, in ungesättigte Harze einpolymerisierbare Arylamin-Derivate, ein Verfahren zu ihrer Herstellung durch Umsetzung von ungesättigten Isocyanaten mit N,N-disubstituierten Arylaminen, wobei wenigstens ein Stickstoffsubstituent eine β-Hydroxyalkylgruppe ist sowie deren Verwendung als Härtungsbeschleuniger für ethylenisch ungesättigte, mit Peroxiden kalt härtbare Harze, wie Acrylate, Methacrylate und ungesättigte Polyesterharze, die sich z. B. zu Spachtel-, Mörtel- oder Zahnfüllmassen verarbeiten lassen.

Die DE-AS 2 308 036 und die DE-OS 2 454 253 beschreiben Monomere, die 1 bis 4 Acryloylgruppen und mindestens 2 Urethangruppen pro Molekül enthalten. Diese Monomeren eignen sich als Ausgangsmaterialien zur Herstellung von Polymerisaten, die aufgrund der Urethangruppen Polyurethanähnliche Eigenschaften besitzen sollen.

Die EP-A 37 314 betrifft thermo- bzw. photopolymerisierbare Zusammensetzungen auf Basis von Umsetzungsprodukten von Hydroxyalkylacrylaten, Lactonen und Isocyanaten. Diese Zusammensetzungen eignen sich als Bindemittel für Tinten, Anstrichmittel und Klebstoffe.

Es ist bekannt, Formmassen auf der Grundlage kalt härtbarer Polyestergießharze unter Verwendung von N,N-Dialkylarylaminen als Polymerisationsbeschleuniger auszuhärten (US-PS 2 480 928). Es ist auch bekannt, ungesättigte Polyester unter Einbau von N,N-Bis-(β-hydroxyalkyl)- arylaminen herzustellen und Formmassen auf dieser Grundlage in Gegenwart von Diacylperoxiden in der Kälte auszuhärten (DE-PS 919 431). Es ist weiterhin bekannt, N,N-Bis-(β-hydroxyalkyl)-arylamine mit Dicarbonsäuren zu einem Polyester oder mit Diisocyanaten zu einem Polyurethan umzusetzen und die resultierenden Produkte ungesättigten Polyesterharzmassen als Härtungsbeschleuniger zuzusetzen (DE-OS 1 943 954, DE-PS 1 643 972). Aus DE-OS 3 202 090, EP-OS 84 784 ist es ebenfalls bekannt, primäre Arylamine in erster Stufe mit Bisepoxiden und in zweiter Stufe mit Monoepoxiden zu oligomeren Härtungsbeschleunigern umzusetzen und diese zur Härtung von ethylenisch ungesättigten Massen zu verwenden.

Die genannten, frei im Harz gelösten Beschleuniger können sowohl vor als auch nach der Härtung migrieren bzw. extrahiert werden oder ihre Reaktivität ist aufgrund ihres polymeren Charakters gering.

Aufgabe der Erfindung ist es, Beschleuniger bereitzustellen, die die oben geschilderten Nachteile nicht aufweisen, die in monomolekularer Form den Harzen zugesetzt und während der Aushärtung in diese eingebaut werden.

Gegenstand der Erfindung sind Verbindungen der Formel I

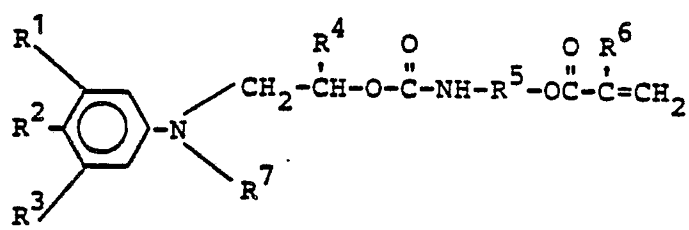

(I)

worin

$R^1$ bis $R^3$ Wasserstoff, $C_1$-$C_{18}$-Alkyl, $C_5$-$C_8$-Cycloalkyl, $C_6$-$C_{14}$-Aryl, Halogen,

$R_4$ Wasserstoff, $C_1$-$C_{18}$-Alkyl, $C_5$-$C_8$-Cycloalkyl,

$R_5$ $C_1$-$C_{18}$-Alkylen, $C_5$-$C_8$-Cycloalkylen, $C_6$-$C_{14}$-Arylen,

$R_6$ Wasserstoff, $C_1$-$C_4$-Alkyl,

$R^7$ einen gesättigten oder ungesättigten, gegebenenfalls OH-substituierten Kohlenwasserstoffrest mit 1-18 C-Atomen darstellen.

Vorzugsweise bedeuten $R^1$ bis $R^4$ und $R^6$ Wasserstoff oder Methyl, $R^5$ $C_2$ bis $C_8$-Alkylen und $R^7$ Methyl. Die Acrylatgruppen der Verbindungen der Formel I sind mit ethylenisch ungesättigten Verbindungen copolymerisierbar.

Die Verbindungen der Formel I werden erhalten, indem man ein tertiäres Amin der Formel II

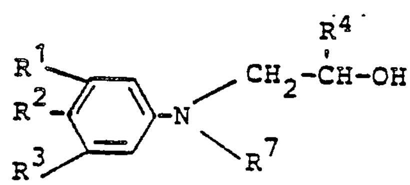

(II)

mit einem Isocyanat der Formel III

$$OCN-R^5-O-\overset{O}{\overset{\|}{C}}-\overset{R^6}{\overset{|}{C}}=CH_2 \quad \text{(III)}$$

worin $R^1$ bis $R^7$ die vorstehend genannten Bedeutungen haben, bei 20 bis 120 °C, vorzugsweise 60-100 °C, umsetzt.

Insbesondere werden equivalente Mengen II und III in Gegenwart eines Schwermetallsalzes, eines tertiären Amins oder eines Alkali- oder Erdalkali-Alkoholates als Katalysator umgesetzt.

Ein Syntheseweg für Isocyanate der Formel (III) ist z. B. in US-PS 2 718 516 und US-PS 2 821 544 beschrieben.

Allgemein können Isocyanate der Formel (III) auch erhalten werden, indem man gegebenenfalls als Säureaddukte vorliegende Dihydrooxazine mit Phosgen bei − 20 bis + 20 °C in einem mit Wasser nicht mischbaren Lösungsmittel in Gegenwart einer wäßrigen Lösung einer Base umsetzt, wobei die Dihydrooxazine die allgemeine Formel

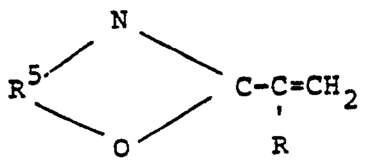

(IV)

aufweisen.

Dihydrooxazine (IV) aus N-Hydroxy-methylamiden der allgemeinen Formel

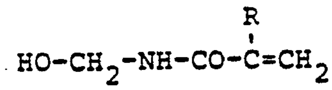

(V)

und einem Olefin können nach dem in Liebigs Annalen *697*, Seiten 171-180 (1966) beschriebenen Verfahren hergestellt werden.

Die Verbindungen der Formel I werden vorzugsweise in einer Menge von 0,008 bis 0,4, insbesondere 0,04 bis 0,2 Gew.-% tertiären Aminstickstoffs, bezogen auf zu härtendes Harz, eingesetzt.

Die Verbindungen der Formel I sind im allgemeinen hochviskose Produkte. Zur besseren Handhabung werden sie — zweckmäßigerweise im Zuge des Herstellungsverfahrens — in geeigneten Lösungsmitteln oder Lösungsmittelgemischen gelöst. Bevorzugte Lösungsmittel sind mit dem Polyester copolymerisierbare Monomere, wie Styrol, α-Methylstyrol oder Ester der Methacrylsäure. Dazu können in begrenztem Umfang polymerisationsinerte Lösungsmittel, wie n-Butylacetat, Cyclohexanon oder Ethylenglykoldimethylether, mitverwendet werden. In dieser Form lassen sich die Beschleuniger den Harzen in jeder Konzentration leicht zudosieren.

Mit den erfindungsgemäßen Verbindungen I härtbare Harze sind alle ethylenisch ungesättigten Verbindungen oder Gemische, die sich in Gegenwart von Diacylperoxiden polymerisieren lassen, vorzugsweise ungesättigte Polyesterharze und Acrylharze.

« Ungesättigte Polyesterharze » sind Mischungen von 30 bis 75 Gewichtsteilen α,β-ethylenisch ungesättigter Polyester und 70 bis 25 Gewichtsteilen damit copolymerisierbarer ungesättigter Monomerer. Sie sind z. B. bie J. R. Lawrence, « Polyester Resins », Reinhold Publ. Corp., New York 1960, S. 18 f., und im Kunststoff-Handbuch, Bd. VIII (« Polyester »), Carl Hanser Verlag, München 1973, S. 247-312, beschrieben.

Bevorzugtes Monomer ist Styrol.

Andere bevorzugte copolymerisierbare Monomere sind im übernächsten Absatz aufgezählt.

« Acrylharze » im Sinne der Erfindung sind (Meth-)-Acryloyloxygruppen enthaltende Polyester, Polyurethane, Polyepoxide, Polyole, Polyetherpolyole. Diese Acrylharze sind bekannt.

Die vorgenannten « Acrylharze » können zur Viskositätserniedrigung, Reaktivitätserhöhung oder zur Erzielung spezieller Eigenschaften auch mit copolymerisierbaren olefinisch ungesättigten Monomeren, z. B. mit (Meth-)Acrylsäureestern einwertiger Alkohole, hydroxyalkyl(meth)-acrylaten, (Meth-)Acrylamiden, Styrol, α-Methylstyrol, durch Alkylgruppen kernsubstituierte Styrole, Divinylbenzol, (Meth-)Acrylnitril, Vinylchlorid, Vinylidenchlorid, Vinylethern, Vinylacetat oder deren Mischungen abgemischt werden. Natürlich ist es auch möglich, mindestens ein α,β-monoolefinisch ungesättigtes Monomeres, beispielsweise der vorstehend angegebenen Art, in Gegenwart der erfindungsgemäßen Verbindungen der Formel I zu polymerisieren.

Vor der Härtung werden den Harzen Polymerisationsinitiatoren, vorzugsweise Diacylperoxide oder Percarbonate, in Mengen von 1 bis 10 Gew.-%, bezogen auf zu härtendes Harz, zugesetzt. Bevorzugte Initiatoren sind z. B. Diacetylperoxid, Dibenzoylperoxid, Di-p-chlorbenzoylperoxid, Phthaloylperoxid, Succinylperoxid, Dilauroylperoxid, Acetylcyclohexansulfonylperoxid, Isopropylpercarbonat, Cyclohexylpercarbonat und Bis-(4-tert.-butylcyclohexyl)-percarbonat.

Die zu härtenden Harzmassen können die üblichen Füllstoffe, Pigmente, Stabilisatoren und Desinfektionsmittel enthalten. Bei Verwendung auf dem Dentalgebiet kommen sowohl organische

Füllstoffe, wie pulverisierte Polyacrylate, als auch anorganische Füllstoffe, wie pulverisiertes Quarz oder Glas bzw. Siliciumdioxid oder Aluminiumoxidpulver, in Frage.

Erfindungsgemäß lassen sich alle Arten von Formteilen in der Kälte aushärten und auf den verschiedensten Gebieten der Bauindustrie, der Elektroindustrie, des Bootsbaus und in der Kraftfahrzeugindustrie einsetzen.

## Beispiele

Herstellung des Beschleunigers 1

Zu einer Mischung aus 1 Mol N-Methyl-N-hydroxyethyl-3,5-dimethylanilin und 1 Mol (1-Isocyanato-2,3-dimethyl)-propylacrylat gab man unter Feuchtigkeitsausschluß 0,1 Gew.-% Dibutylzinndilaurat und rührt bei 60 °C 6 h lang.

IR ($cm^{-1}$) : keine Isocyanat-Absorption bei 2275-2250, Absorption bei 3350, 1730, 1710, 1640.

Herstellung des Beschleunigers 5

Entsprechend vorstehender Methode wurden 1 Mol N,N-Bis-(β-hydroxyethyl)-p-toluidin und 2 Mol (1-Isocyanato)-ethylmethacrylat umgesetzt.

IR ($cm^{-1}$) : keine Isocyanat-Absorption bei 2275-2250, Absorption bei 3350, 1730, 1680, 1640.

Die Beschleuniger 2, 3 und 4 wurden analog 1 und 2 aus 1 Mol N-Methyl-N-(2-hydroxypropyl)-3,5-dimethylanilin und 1 Mol (1-Isocyanato-2,3-dimethyl)-propylacrylat (2), 1 Mol N-Methyl-N-(2-hydroxypropyl)-3,5-dimethylanilin und 1 Mol (1-Isocyanato)-ethylmethacrylat (3) und 1 Mol N,N-Bis-(2-hydroxypropyl)-3,5-dimethylanilin und 2 Mol (1-Isocyanato)-ethylmethacrylat (4) hergestellt.

Beschleuniger A

Beschleuniger A ist ein Polykondensationsprodukt aus N,N-Bis-(2-hydroxypropyl)-p-toluidin und Adipinsäure (DE-OS 3 202 090).

Herstellung von ungesättigten Polyesterharzen

Polyesterharz I

Aus 89 Mol Diethylenglykol, 13 Mol Ethylenglykol, 98,1 Mol Maleinsäureanhydrid und 42,1 Mol Dicyclopentadien wurde durch Schmelzkondensation ein Polyester hergestellt. Anschließend wurde das Harz zu einer 63 gew.-%igen Lösung in Styrol gelöst und mit 0,04 Gew.-% Chloranil und 0,01 Gew.-% Kupfernaphthenat, jeweils bezogen auf das Polyesterharz, stabilisiert. Das Polyesterharz hatte eine Viskosität von 480 mPas (gemessen bei 25 °C) und eine Säurezahl von 10.

Polyesterharz II

Polyesterharz II ist ungesättigtes Polyesterharz aus 2 981 kg Maleinsäureanhydrid, 1 930 kg Phthalsäureanhydrid, 2 121 kg Diethylenglykol und 1 938 kg Propylenglykol 60 gew.-%ig in Styrol mit einer Viskosität von 270-370 m Pa.s, einer Iodzahl kleiner als 2 und einer Dichte von 1,1 $g/cm^3$.

Bestimmung der Reaktivität

Mit den Beschleunigern 1 bis 5 und A und den Polyesterharzen I und II wurden kalt härtbare Gießharzmassen hergestellt. Der bei den angegebenen Konzentrationen gegebene Gehalt an aromatisch gebundenem Stickstoff in dem kalthärtenden Harz ermöglicht einen Vergleich der Reaktivität der einzelnen Beschleuniger. Die Reaktivität wurde bestimmt, indem die Beschleuniger/Harzmischung bei einer Anfangstemperatur von 25 °C mit 2 % handelsüblicher Benzoylperoxid-Paste (50 % Benzoylperoxid-Gehalt) ausgehärtet wurde.

Die Bestimmung der Gelzeit, Härtezeit und Maximaltemperatur erfolgte gemäß DIN 16 945.

Härtung in Polyesterharz I

| Beschleuniger | % im Harz | % N im Harz | Gelzeit (min) | Härtzeit (min) | max. Temp. (°C) |
|---|---|---|---|---|---|
| 1 | 1,58 | 0,064 | 5,9 | 8,8 | 110 |
| 2 | 1,65 | 0,064 | 6,8 | 9,8 | 110 |

(Fortsetzung)

| Beschleuniger | % im Harz | % N im Harz | Gelzeit (min) | Härtzeit (min) | max. Temp. (°C) |
|---|---|---|---|---|---|
| 3 | 1,58 | 0,064 | 5,8 | 8,6 | 103 |
| 4 (Vergleich) | 2,20 | 0,064 | 25,6 | 32,8 | 105 |
| 5 (Vergleich) | 2,08 | 0,064 | 24,0 | 30,2 | 118 |
| A (Vergleich) | 2,0 | 0,064 | 11,0 | 17,2 | 110 |
| Härtung in Polyesterharz II | | | | | |
| 2 | 1,55 | 0,060 | 3,2 | 5,9 | 141 |
| 5 (Vergleich) | 1,96 | 0,060 | 8,8 | 11,7 | 153 |
| A (Vergleich) | 1,85 | 0,060 | 4,3 | 7,0 | 144 |

**Patentansprüche**

1. Verbindungen der Formel I

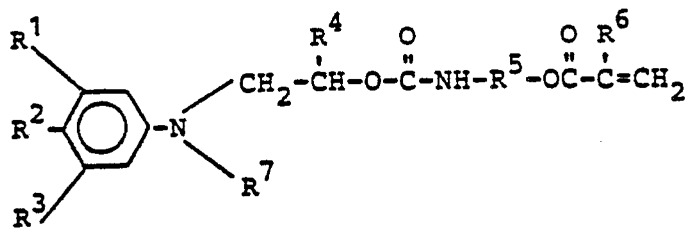

(I)

worin

$R^1$ bis $R^3$ Wasserstoff, $C_1$-$C_{18}$-Alkyl, $C_5$-$C_8$-Cycloalkyl, $C_6$-$C_{14}$-Aryl, Halogen,
$R^4$ Wasserstoff, $C_1$-$C_{18}$-Alkyl, $C_5$-$C_8$-Cycloalkyl,
$R^5$ $C_1$-$C_{18}$-Alkylen, $C_5$-$C_8$-Cycloalkylen, $C_6$-$C_{14}$-Arylen,
$R^6$ Wasserstoff, $C_1$-$C_4$-Alkyl,
$R^7$ einen gesättigten oder ungesättigten, gegebenenfalls OH-substituierten Kohlenwasserstoffrest mit 1-18 C-Atomen darstellen.

2. Verbindungen nach Anspruch 1, worin $R^1$ bis $R^4$ und $R^6$ Methyl oder Wasserstoff, $R^5$ $C_2$-$C_8$-Alkylen und $R^7$ Methyl bedeuten.

3. Verfahren zur Herstellung von Verbindungen gemäß Anspruch 1, dadurch gekennzeichnet, daß man ein tertiäres Amin der Formel II

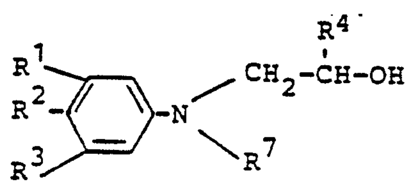

(II)

mit einem Isocyanat der Formel III

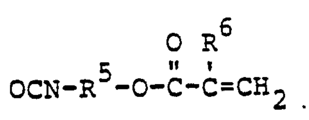

(III)

worin $R^1$ bis $R^7$ die in Anspruch 1 genannten Bedeutungen haben, bei 20 bis 120 °C umsetzt.

4. Verfahren nach Anspruch 3, dadurch gekennzeichnet, daß die Verbindungen II und III in äquivalenten Mengen in Gegenwart eines Schwermetalles, eines tertiären Amins oder eines Alkali- oder Erdalkali-alkoholates als Katalysator umgesetzt werden.

5. Verwendung der Verbindungen nach Anspruch 1 als einbaubarer Härtungsbeschleuniger für ethylenisch ungesättigte, kalt härtbare Harze.

**Claims**

1. Compounds of the formula I

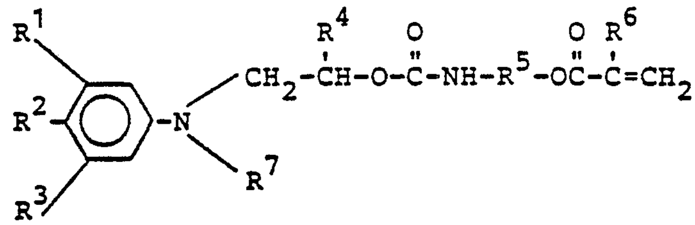

(I)

wherein
$R^1$ to $R^3$ represent hydrogen, $C_1$-$C_{18}$-alkyl, $C_5$-$C_8$-cycloalkyl, $C_6$-$C_{14}$-aryl or halogen,
$R^4$ represents hydrogen, $C_1$-$C_{18}$-alkyl or $C_5$-$C_8$-cycloalkyl,
$R^5$ represents $C_1$-$C_{18}$-alkylene, $C_5$-$C_8$-cycloalkylene or $C_6$-$C_{14}$-arylene,
$R^6$ represents hydrogen or $C_1$-$C_4$-alkyl and
$R^7$ represents a saturated or unsaturated optionally OH-substituted hydrocarbon radical having 1-18 C atoms.

2. Compounds according to Claim 1, wherein $R^1$ to $R^4$ and $R^6$ denote methyl or hydrogen, $R^5$ denotes $C_2$-$C_8$-alkylene and $R^7$ denotes methyl.

3. Process for the preparation of compounds according to Claim 1, characterised in that a tertiary amine of the formula II

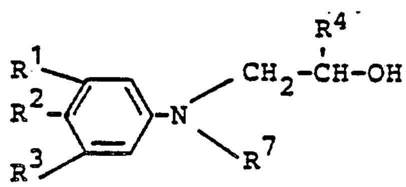

(II)

is reacted with an isocyanate of the formula III

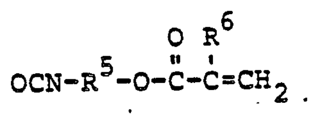

(III)

wherein $R^1$ to $R^7$ have the meanings stated in Claim 1, at 20 to 120 °C.

4. Process according to Claim 3, characterised in that the compounds II and III are reacted in equivalent amounts, in the presence of a heavy metal, of a tertiary amine or of an alkali metal alcoholate or alkaline earth metal alcoholate as a catalyst.

5. Use of the compounds according to Claim 1 as curing accelerators which can be incorporated into ethylenically unsaturated, cold-curable resins.

**Revendications**

1. Composés de formule (I)

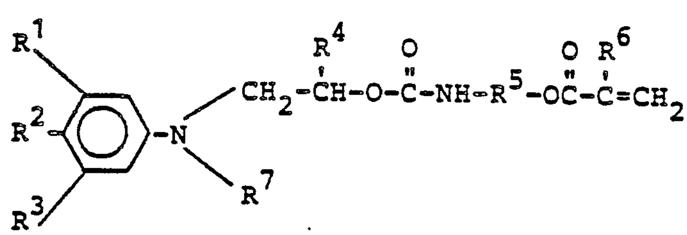

(I)

dans laquelle
$R^1$ à $R^3$ représentent chacun un atome d'hydrogène, un groupe alkyle en $C_1$ à $C_{18}$, un groupe cycloalkyle en $C_5$ à $C_8$, un groupe aryle en $C_6$ à $C_{14}$, un atome d'halogène,
$R^4$ représente un atome d'hydrogène, un groupe alkyle en $C_1$ à $C_{18}$, un groupe alkyle en $C_5$ à $C_8$,

$R^5$ représente un groupe alkylène en $C_1$ à $C_{18}$, un groupe cycloalkylène en $C_5$ à $C_8$, un groupe arylène en $C_6$ à $C_{14}$,

$R^6$ représente un atome d'hydrogène ou un groupe alkyle en $C_1$ à $C_4$,

$R^7$ représente un reste d'hydrocarbure saturé ou insaturé, éventuellement substitué par OH, comportant 1 à 18 atomes de carbone.

2. Composés selon la revendication 1, dans lesquels $R^1$ à $R^4$ et $R^6$ représentent chacun un groupe méthyle ou un atome d'hydrogène, $R^5$ représente un groupe alkylène en $C_2$ à $C_8$ et $R^7$ représente un groupe méthyle.

3. Procédé pour préparer des composés selon la revendication 1, caractérisé en ce qu'on fait réagir à 20 jusqu'à 120 °C une amine tertiaire de formule (II)

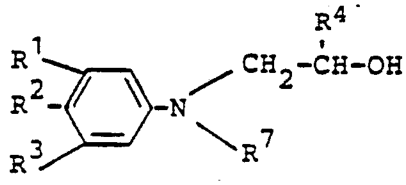

(II)

avec un isocyanate de formule (III)

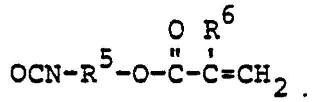

(III)

où $R^1$ à $R^7$ ont les sens indiqués à la revendication 1.

4. Procédé selon la revendication 3, caractérisé en ce qu'on fait réagir les composés II et III en des quantités équivalentes en présence d'un métal lourd, d'une amine tertiaire ou d'un alcoolate alcalin ou alcalino-terreux comme catalyseur.

5. Utilisation des composés selon la revendication 1 comme composés incorporables à des résines à insaturation éthylénique, durcissables à froid, pour accélérer le durcissement de ces résines.